# EUROPEAN PATENT APPLICATION

(11) **EP 4 137 170 A1**
(43) Date of publication of application: **22.02.2023**
(21) Application number: 21020391.5
(22) Date of filing: 29.07.2021
(51) Int. Cl.: A61M 1/16

(54) **PURGE FLUID GENERATOR FOR MEMBRANE OXYGENATION**

(71) Applicant: Bast, Tim, 247 31 Södra Sandby (SE)
(72) Inventor: Bast, Tim, 247 31 Södra Sandby (SE)

(57) **Abstract**

The present invention provides a gas composition for use in preparation of a purge fluid for membrane oxygenation. In another aspect, the present invention provides a purge fluid generator for supplying a purge fluid to a membrane oxygenation device which has been prepared using the gas composition. In another aspect, the present invention provides a pressure vessel for use in in a purge fluid generator for supplying purge fluid to a membrane oxygenation device. The pressure vessel contains the gas composition. In another aspect, the present invention provides a method for conditioning the oxygenation membrane in extracorporeal membrane oxygenation using the gas composition.

## Description

### TECHNICAL FIELD

The present invention relates to purge fluids, more specifically to purge fluids for being supplied to a membrane oxygenation device. The present invention further relates to a purge fluid generator, more specifically to a purge fluid generator for supplying purge fluid to a membrane oxygenation device. The present invention further relates to a pressure vessel for use in a purge fluid generator for supplying purge fluid to a membrane oxygenation device. The present invention further relates to a method for conditioning the oxygenation membrane in extracorporeal membrane oxygenation

### BACKGROUND

In intensive care medicine, it is often necessary to support the blood oxygenation of a patient with external oxygenation devices during an acute respiratory stress syndrome, i.e., when the regular oxygenation through the patient's lungs has deteriorated below a critical level.

Extracorporeal membrane oxygenation (ECMO) devices have been used for longer term external blood oxygenation such as for periods of three to ten days. ECMO devices typically include a membrane for exchanging oxygen and carbon dioxide between a first volume through which the patient's blood flows and a second volume into which a purge fluid (also referred to as sweep gas) is supplied. The membrane is impermeable for blood but permeable for oxygen and carbon dioxide. The effectiveness of the blood oxygenator depends on the available surface area of the membrane through which the gases can be exchanged as well as on the difference in partial pressures of oxygen and carbon dioxide between the blood and the purge fluid on the other side of the membrane.

One challenge in ECMO is that the large internal surface area needed for the gas exchange in the membrane oxygenator activates the platelets, leading to coagulation inside the membrane pores. Over time, this clot formation leads to clogging of the membrane pores and shortens the lifetime of the membrane oxygenator. The oxygenator membrane is typically arranged in a cartridge which allows convenient replacement since regular exchanges are foreseen in state of the art ECMO devices. Since every exchange of the oxygenator cartridge is disruptive and increases the risk of patient complications, it is desirable to recduce the clotting as much as possible to enhance the useful lifespan of each oxygenator cartridge.

Nitric oxide is known as an inhaled gas medication which mitigates the risk of hypoxic respiratory failure by acting as a vasodilator. The medication is typically used in conjunction with a ventilator or with a nasal cannula. It is mixed into the inspiratory limb of the breathing circuit at a concentration of around 20 volume-PPM for neonates.

### SUMMARY OF THE INVENTION

The object of the present invention is to overcome the problems posed by the existing devices and methods for membrane oxygenation.

One specific object of the present invention is to prolong the timespan during which membrane oxygenator cartridges can be used before it needs to be replaced.

Another specific object is to reduce platelet adhesion to the membrane surface of a membrane oxygenation device.

Another specific object is to reduce platelet coagulation in the vicinity of the membrane surfaces such as in the membrane pores
Another specific object of the present invention is the reduction of thromboembolic complications in patients supported by extracorporeal membrane oxygenation.

Another specific object of the present invention is to reduce the occurrences of using nitric oxide as an inhaled gas medicament being necessary.

Another specific object of the present invention is to reduce the required amount of anti-coagulants such as heparin in patients supported by extracorporeal membrane oxygenation.

Another specific object of the present invention is the reduction of haemorrhagic complications in patients supported by extracorporeal membrane oxygenation.

Another specific object of the present invention is to make extracorporeal membrane oxygenation available to patients who do not tolerate high levels of anti-coagulants such as heparin due to other potentially life-threatening conditions such as intracranial haemorrhage.

In one aspect, the present invention provides a gas composition for use in preparation of a purge fluid for membrane oxygenation wherein the gas composition comprises at least 3'000 volume-PPM of nitric oxide.

In another aspect, the present invention provides a purge fluid generator for supplying a purge fluid to a membrane oxygenation device. The purge fluid generator comprises a first gas supply unit, a second gas supply unit, a purge fluid outlet, and a mixing unit. A first inlet of the mixing unit is connected to the first gas supply unit and a second inlet of the mixing unit is connected to the second gas supply unit. The first gas supply unit is configured to supply a gas mixture comprising at least one gas selected from the group of air, oxygen, nitrogen, or carbon dioxide. The second gas supply unit is configured to supply the gas composition for use in preparation of a purge fluid for membrane oxygenation according to the present invention. The mixing unit is configured to deliver a purge fluid comprising between 1 and 200 volume-PPM of nitric oxide into the purge fluid outlet.

In another aspect, the present invention provides a pressure vessel for use in in a purge fluid generator for supplying purge fluid to a membrane oxygenation device. The pressure vessel has a volume of less than 5 litres and contains the gas composition for use in preparation of a purge fluid for membrane oxygenation according to the present invention.

In another aspect, the present invention provides a method for conditioning the oxygenation membrane in extracorporeal membrane oxygenation. The method comprises the step of receiving a purge fluid for membrane oxygenation comprising at least one of the following: oxygen, nitrogen, carbon dioxide, air. The method further comprises the step of supplying a gas composition comprising nitric oxide. The method further comprises the step of mixing the gas composition into the purge fluid for membrane oxygenation. The method further comprises the step of exposing one surface of the oxygenation membrane to the purge fluid mixed with the gas composition.

Further advantageous embodiments of the gas composition, the purge fluid generator, the pressure vessel, and the method for conditioning the oxygenation membrane of the present invention are specified in the dependent claims.

### BRIEF DESCRIPTION OF THE FIGURES

Non-limiting embodiments of the present invention will be described by way of example with reference to the accompanying drawings, in which:
Figure 1 shows a schematic representation of the purge fluid generator according to the present invention.
Figures 2 through 8 shows schematic representations of various exemplary non-limiting purge fluid generators according to the present invention with various optional additional features and characteristics.
Figure 9 shows a schematic representation of the method for conditioning the oxygenation membrane in extracorporeal membrane oxygenation according to the present invention.

### DETAILED DESCRIPTION OF THE INVENTION

The present invention provides a purge fluid for being supplied to a membrane oxygenation device.

The term **"membrane oxygenation device"** as used herein refers to a device comprising a gas-permeable membrane having a first and a second surface across which oxygen and carbon dioxide may be exchanged. The membrane is impermeable for fluids such as blood so that fluids cannot permeate across the membrane from outside the first surface to outside of the second surface. One principal mode of operation of a membrane oxygenation device is to oxygenate blood and to remove carbon dioxide from the blood in longer term life support, termed extracorporeal membrane oxygenation (ECMO).

Suitable gas-permeable membranes include without limitation the membranes used in state-of-the-art oxygenators such as the *Quadrox-i* family of oxygenators commercially available from Getinge. Various spatial arrangements of the gas permeable membrane are suitable for use with the present invention such as planar, cylindrical, spherical, and combinations thereof. A preferred configuration is the configuration of a hollow fibre in which the first surface is facing inward towards the internal volume of the hollow fibre and the second surface is facing outward. The blood may flow through the internal volume of the hollow fibre.

The present invention provides a purge fluid for being supplied to a membrane oxygenation device.

The term **"purge fluid"** as used herein refers to a fluid which is suitable supplying oxygen to blood and accepting carbon dioxide across the gas-permeable membrane by means of a difference in partial pressure. The purge fluid may be a purge gas.

The purge fluid may flow along the second surface of the membrane oxygenation device while the blood is flowing along the first surface. The first surface may be the inner surface of a hollow fibre membrane and the second surface may be the outer surface of a hollow fibre membrane. One suitable type of hollow fibre is made from polymethylpentene and widely used in commercially available membrane oxygenation devices.

The present invention provides a gas composition which is useful in the preparation of a purge fluid for membrane oxygenation in a membrane oxygenation device. The gas composition comprises at least 3'000 volume-PPM of nitric oxide (chemical formula NO), preferably at least 6'000 volume-PPM, more preferably at least 8'000 volume-PPM, yet more preferably at least 10'000 volume-PPM.

For handling and storage in a pressure vessel prior to being dispensed, the nitric oxide gas may be mixed with another gas which is inert to reacting with nitric oxide, will not cause the nitric oxide to react, decompose, or convert to other molecules comprising nitrogen and oxygen, and suitable for being mixed with other components into a purge fluid. A suitable gas for being mixed with nitric oxide is nitrogen because nitrogen is a component of regular air and also of conventional purge fluids.

In another aspect, the present invention provides a purge fluid generator for supplying a purge fluid to a membrane oxygenation device. The composition of the purge fluid is adapted for being suitable for membrane oxygenation. The specific composition of the purge fluid is dependent on patient supported by the membrane oxygenation device as well as on the patient's condition. The specific composition generally needs to be initially defined and then regularly adjusted on a case-by-case basis based on medical considerations. The purge fluid generator comprises a first gas supply unit and a second gas supply unit. The purge fluid generator further comprises a mixing unit and a purge fluid outlet. The mixing unit has a first inlet and a second inlet. The first inlet is connected to the first gas supply unit and the second inlet is connected to the second gas supply unit. The mixing unit is configured to mix the gas received from the first gas supply unit and the gas received from the second gas supply unit. The mixing unit is be configured to deliver a purge fluid to the purge fluid outlet.

The first gas may be a gas composition comprising at least one gas selected from the group of air, oxygen, nitrogen, or carbon dioxide. This gas composition may generally correspond to the purge fluids used in known cardiopulmonary bypasses and a membrane oxygenation devices. In particular, the desired fraction of inhaled oxygen in the purge fluid depends strongly on the patient and the patient's condition. The fraction of inhaled oxygen and may be adjustable such that it can be adjusted by the caretaker or automatically by the purge fluid generator accordingly.

The second gas may be the gas composition for use in preparation of a purge fluid for membrane oxygenation of the present invention.

The first gas supply unit may be configured such that the proportion of the first and second gas components can be adjusted. The adjustment can be done manually be the operator or automatically through a control unit comprised in the first gas supply unit. The control unit of the first gas supply unit may be connected to a control unit in the purge fluid generator. The second gas supply unit may be configured such that the proportion of the gas components can be adjusted. The adjustment can be done manually be the operator or automatically through a control unit comprised in the second gas supply unit. The control unit of the second gas supply unit may be connected to a control unit in the purge fluid generator.

The purge fluid is obtained by mixing the first gas and the second gas. The first gas and the second gas are mixed in the mixing unit. The mixing unit may be mechanically adjustable or electronically adjustable. The purge fluid generator may comprise a first sensor such as a mass flow sensor or a volume flow sensor for measuring the gas flow from the first gas supply unit to the mixing unit. The purge fluid generator may comprise a second sensor such as a mass flow sensor or a volume flow sensor for measuring the gas flow from the second gas supply unit to the mixing unit. The purge fluid generator may comprise two sensors such as two mass flow sensors or two volume flow sensors for measuring the gas flow from both the first gas supply unit and the second gas supply unit to the mixing unit. The purge fluid generator may comprise a first adjustable valve such as a control valve or a proportional valve for controlling the gas flow from the first gas supply unit to the mixing unit. The purge fluid generator may comprise a second adjustable valve such as a control valve or a proportional valve for controlling the gas flow from the second gas supply unit to the mixing unit.

The purge fluid generator may comprise a control unit. The first and/or second sensor may be electronically connected to the control unit. The control unit may be configured to receive from the first sensor a first signal indicative of the gas flow from the first gas supply unit to the mixing unit. The control unit may be configured to receive from the second sensor a second signal indicative of the gas flow from the second gas supply unit to the mixing unit. The control unit may be configured to operate any of the control valves by providing signals for opening and closing the control valves in adjustable intervals to regulate the gas flow through the valve. The control unit may be configured to apply fixed time intervals for opening the control valve and adjust the time lapse between the opening intervals. A preferred switching time for any of the control valves is 2 milliseconds or less. A suitable open cycle for any of the control valves is between 200 and 500 milliseconds, preferably about 300 milliseconds. The control unit may operate any of the proportional valves by providing an adjustable control signal indicative of the desired flow rate. The first adjustable valve and/or the second adjustable valve may take the form an array of control valves with increasing diameter flow channels to address a larger interval of desired flow rates.

The first and/or second adjustable valve may be electronically connected to the control unit. The control unit may be configured to control the composition of the purge fluid by adjusting the first and the second adjustable valves. The control unit may be configured to control the gas flow from the first gas supply unit or from the second gas supply unit or from both to maintain the relative concentrations of the first and second gases at a predetermined level. The control unit may be configured to keep the relative concentrations constant over time or to adjust the relative concentrations according to a cyclic or other pattern which may be correlated to the medically desired ventilation breathing pattern of the patient.

The purge fluid generator may be configured to deliver a constant flow of purge fluid to the purge fluid outlet. The purge fluid generator may be configured to deliver a varying flow of purge fluid to the purge fluid outlet. The variation of the purge fluid outlet may follow a cyclic or other pattern which may be correlated to the medically desired ventilation pattern of the patient. The mixing unit may be configured to deliver a purge fluid comprising less than 200 volume-PPM of nitric oxide into the purge fluid outlet. The mixing unit may be configured to deliver a purge fluid comprising more than 1 volume-PPM of nitric oxide into the purge fluid outlet. The mixing unit may be configured to deliver a purge fluid comprising between 1 and 200 volume-PPM of nitric oxide into the purge fluid outlet. The mixing unit may be configured to deliver a purge fluid comprising an adjustable concentration of nitric oxide into the purge fluid outlet. The control unit may be configured to control the concentration of nitric oxide comprised in the purge fluid. The control unit may be configured to adjust the concentration of nitric oxide in accordance with a periodic or other pattern which is correlated to extending the useful lifetime of the oxygenator membrane.

The first gas supply unit may comprise a third gas supply unit and a fourth gas supply unit together with a first gas supply outlet. The first gas supply unit may further comprise a fifth gas supply unit. The third gas supply unit may be configured to supply oxygen. The fourth gas supply unit may be configured to supply air or the fourth gas supply unit may be configured to supply nitrogen. The fifth gas supply unit may be configured to supply carbon dioxide. The first gas supply unit may comprise a second mixing unit for mixing the gas which is received from the third gas supply unit and the fourth gas supply unit and optionally from the fifth gas supply unit. A first inlet of the second mixing unit may be connected to the outlet of the third gas supply unit and a second inlet of the second mixing unit may be connected to the outlet of the fourth gas supply unit. A third inlet of the second mixing unit may be connected to the outlet of the fifth gas supply unit. An outlet of the second mixing unit may be connected to the first gas supply outlet of the first supply unit. The first gas supply outlet may be connected to the first inlet of the mixing unit.

The first gas supply unit may further comprise a third adjustable valve such as a control valve or a proportional valve for controlling the gas flow from the third gas supply unit to the second mixing unit. The first gas supply unit may further comprise a third sensor such as a mass flow sensor or a volume flow sensor for measuring the gas flow from the third gas supply unit to the second mixing unit. The first gas supply unit may further comprise a fourth adjustable valve such as a control valve or a proportional valve for controlling the gas flow from the fourth gas supply unit to the second mixing unit. The first gas supply unit may further comprise a fourth sensor such as a mass flow sensor or a volume flow sensor for measuring the gas flow from the fourth gas supply unit to the second mixing unit. The first gas supply unit may further comprise a fifth adjustable valve such as a control valve or a proportional valve for controlling the gas flow from the fifth gas supply unit to the second mixing unit. The first gas supply unit may further comprise a fifth sensor such as a mass flow sensor or a volume flow sensor for measuring the gas flow from the fifth gas supply unit to the second mixing unit. The first gas supply unit may comprise a control unit. The control unit may be electronically connected to at least one of the third sensor, the fourth sensor, the fifth sensor, the third adjustable valve, the fourth adjustable valve, or optionally the fifth adjustable valve. The control unit may be configured two adjust the relative concentrations off the third gas, the fourth gas, and the fifth gas by adjusting the third adjustable valve, the fourth adjustable valve, and optionally the fifth adjustable valve. The control unit may be configured to keep the concentrations constant overtime or to adjust the relative concentrations according to a cyclic or other pattern which is correlated two the medically desired ventilation pattern of the patient.

The third gas supply unit may comprise a gas inlet which is configured to receive the third gas from an external gas supply such as a wall dispenser in a patient room or operating theatre. The fourth gas supply unit may comprise against inlet which is configured to receive the fourth gas from an external gas supply such as a wall dispenser in the patient room or operating theatre. The fifth gas supply unit may comprise a gas inlet which is configured to receive the fifth gas from an external gas supply such as a wall dispenser in the patient room or operating theatre.

The purge fluid generator may further comprise a pressure sensor for measuring the pressure of the pressure of the purge fluid at the outlet of the mixing unit. The pressure sensor may be electronically connected to the control unit for receiving a signal indicative of the pressure of the purge fluid at the outlet of the mixing unit. The control unit may be configured to control the pressure of the purge fluid at the outlet of the mixing unit in response to the pressure sensed by the pressure sensor. The control unit may be configured to initiate an alarm signal in case the pressure off the purge fluid at the outlet of the mixing unit is too high for safe ventilation of the patient.

The purge fluid generator may further comprise an output flow sensor such as a mass flow sensor or a volume flow sensor configured for measuring the gas flow of purge fluid at the outlet of the mixing unit. The output flow sensor may be electronically connected to the control unit of the purge fluid generator for receiving a signal indicative of the gas flow of the purge fluid. The control unit may be configured to control the gas flow of the purge fluid at the outlet of the mixing unit. The control unit may be configured to control the connected valves so that the purge fluid generator delivers a constant flow of purge fluid at the outlet of the mixing unit. The control unit may be configured to control the connected valves so that the purge fluid generator delivers a cyclic or other flow pattern of purge fluid at the outlet of the mixing unit whereby the cyclic or other pattern is correlated to the medically desired ventilation pattern of the patient.

The purge fluid generator may further comprise a concentration sensor for measuring the nitric oxide partial pressure in the purge fluid at the outlet of the mixing unit. The concentration sensor may be connected to the control unit. The control unit may be configured to control the nitric oxide partial pressure in the purge fluid at the outlet of the mixing unit in response to the concentration sensed by the concentration sensor. The possibility to measure the concentration of the nitric oxide at the outlet of the mixing unit provides the opportunity for the control unit to adjust the valves such that the concentration of nitric oxide is kept constant or in accordance with a cycling pattern.

The control unit of the purge fluid generator may be configured to receive one or more sensor signals indicative of: oxygen partial pressure of the purge fluid flowing to the membrane oxygenation device, carbon dioxide partial pressure of the purge fluid flowing to the membrane oxygenation device, oxygen partial pressure of the purge fluid emitted from the membrane oxygenation device, carbon dioxide partial pressure of the purge fluid emitted from the membrane oxygenation device, oxygen partial pressure of the purge fluid flowing to a ventilator connected to the same patient as the membrane oxygenation device, carbon dioxide partial pressure of the purge fluid flowing to the ventilator, oxygen partial pressure of the purge fluid emitted from the ventilator, carbon dioxide partial pressure of the purge fluid emitted from the ventilator, water vapor partial pressure of the purge fluid emitted from the membrane oxygenation device, and nitric oxide or nitrogen dioxide in the oxygenated return blood of the extracorporeal membrane oxygenation apparatus. These one or more parameters can be important in monitoring the effectiveness of the oxygenation of and the removal of carbon dioxide from the blood of the patient. In turn, the effectiveness of the patient oxygenation can be an indicator for the status and remaining useful lifetime of the oxygenation membrane. The control unit may be configured to adjust the relative concentrations of the individual gases contained in the purge fluid in response to the one or more sensor signals received by the control unit. The control unit may be configured to adjust the gas flow and or the gas pressure of the purge fluid at the outlet of the mixing unit in response to the one or more sensor signals received. The control unit may be configured to display or play back an alarm signal in response to the one or more sensor signals received. The control unit may be configured to estimate the remaining useful lifetime of the membrane oxygenation unit based on the one or more sensor signals received. The control unit may be configured to compute the change rate of any of the received sensor signals.

The second gas supply unit may comprise a pressure vessel filled with the gas composition for use in preparation of a purge fluid for membrane oxygenation according to the present invention. The pressure vessel preferably has a volume of no more than 5 litres, more preferably no more than 3 litres, yet more preferably no more than 2 litres. A small pressure vessel is usable in an environment that has limited space for equipment such as an operating theatre, a patient transport vehicle such as an ambulance or a helicopter, or a patient room in which the space around the patient must be shared with other equipment. The concentration of nitric oxide in the pressure vessel is preferably at least 3'000 volume-PPM, yet more preferably at least 6'000 volume-PPM, yet more preferably at least 8000 volume-PPM, yet more preferably at least 10'000 volume-PPM. A higher concentration of nitric oxide corresponds to a higher amount off nitric oxide in a pressure vessel off given volume. Higher concentrations allow a longer use time before the pressure vessel must be exchanged. Such an extended use time allows for less frequent interruptions of the patient treatment or for a prolonged treatment or transport of the patient without having to carry replacement pressure vessels.

The purge fluid generator may be configured for mobile use. The mobility is important in situations where the patient must be transported between different rooms in the hospital or even between hospitals. The mobility of the purge fluid generator would further allow to carry such a mobile unit as part of the standard equipment in emergency rescue vehicles. The purge fluid generator may be configured for mobile use by placing all components within the same frame or housing. The purge fluid generator may be configured for mobile use by placing it in the same housing with a mobile ECMO unit. The purge fluid generator may be configured for mobile use by building it so that it can withstand external forces or vibrations or changes in external conditions. The purge fluid generator may be configured for mobile use by being equipped with magnetic shielding so it can be used while the patient is being exposed to high magnetic fields such as during NMR imaging. The purge fluid generator may be configured for mobile use by comprising an independent internal power supply unit, preferably having sufficient capacity for supplying at least 120min of power for operating the purge fluid generator independently from an external power supply, more preferably for supplyng at least 300min of power.

In the purge fluid generator of the present invention, the fluid conduits for channelling the fluid flows may be made from stainless steel, or polyoxymethylene, or any other material that is suitable to withstand being exposed to fluids having a nitric oxide concentration of 10'000 ppm, preferably suitable to withstand being exposed to fluids having a nitric oxide concentration of 20'000 ppm or more.

Flow sensors suitable for the purge fluid generator of the present invention include without limitation flow sensor based on the principle of hot-wire anemometry, differential pressure, ultrasound reflection, thermal mass flow, and mass flow.

In a further aspect, the present invention provides a method for conditioning the oxygenation membrane in extracorporeal membrane oxygenation. The method comprises the step of receiving a purge fluid for membrane oxygenation comprising at least one of the following: oxygen, nitrogen, carbon dioxide, air. The method further comprises the step of supplying a gas composition comprising nitric oxide. The method further comprises the step of mixing the gas composition into the purge fluid for membrane oxygenation. The method further comprises the step of exposing one surface of the oxygenation membrane to the purge fluid mixed with the gas composition. The gas composition may comprise at least 3'000 volume-PPM of nitric oxide, preferably at least 6'000 volume-PPM, more preferably at least 8000 volume-PPM, yet more preferably at least 10'000 volume-PPM.

The method for conditioning the oxygenation membrane in extracorporeal membrane oxygenation may further comprise the steps of: monitoring the nitric oxide or nitrogen dioxide concentration in the oxygenated return blood of the extracorporeal membrane oxygenation apparatus and adjusting the nitric oxide concentration in the purge fluid in response to the nitric oxide or nitrogen dioxide concentration in the oxygenated return blood of the extracorporeal membrane oxygenation apparatus. Monitoring the nitric oxide or nitrogen dioxide in the oxygenated return blood is important for ascertaining that the patient is not exposed to undesired levels of nitric oxide while the oxygenation membrane is conditioned applying this method.

The method for conditioning the oxygenation membrane in extracorporeal membrane oxygenation may be already initiated or carried out before the patient is connected to the extracorporeal membrane oxygenation device. In this case, the membrane surfaces are conditioned at least once before the start of the blood oxygenation. During the blood oxygenation, the addition of the nitric oxide may be reduced or completely stopped. The method for conditioning the oxygenation membrane in extracorporeal membrane oxygenation may be repeated for one or more defined periods during the blood oxygenation but reduced or stopped otherwise.

### DETAILED DESCRIPTION OF THE EXAMPLES

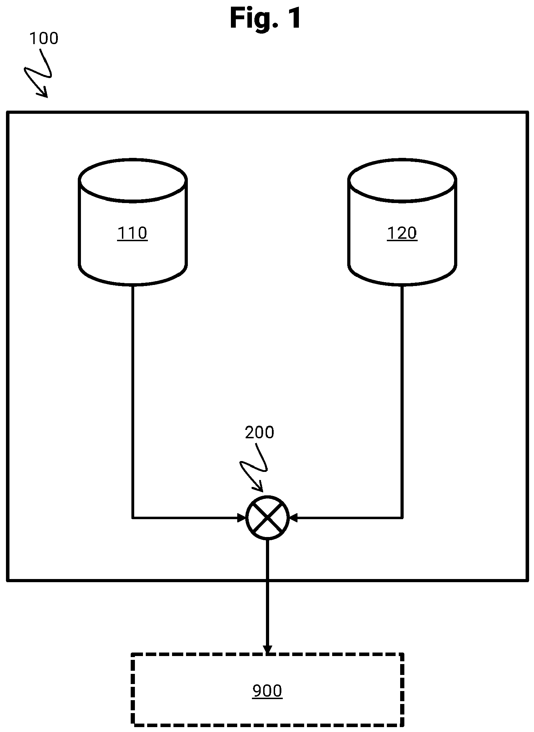

Figure 1 shows the purge fluid generator 100 for supplying a purge fluid to a membrane oxygenation device 900 of the present invention. The membrane oxygenation device 900 itself is not part of the present invention. The purge fluid generator comprises a first gas supply unit 110 at a second gas supply unit 120. The purge fluid generator 100 comprises mixing unit 200. One inlet of the mixing unit 200 is connected to the first gas supply unit 110. Another inlet of the mixing unit 200 is connected to the second gas supply unit 120. The outlet of the mixing unit 200 is configured to be connected to the membrane oxygenation device 900. The first gas supply unit 110 at the second gas supply unit 120 and the mixing unit 200 are optionally placed within the same housing.

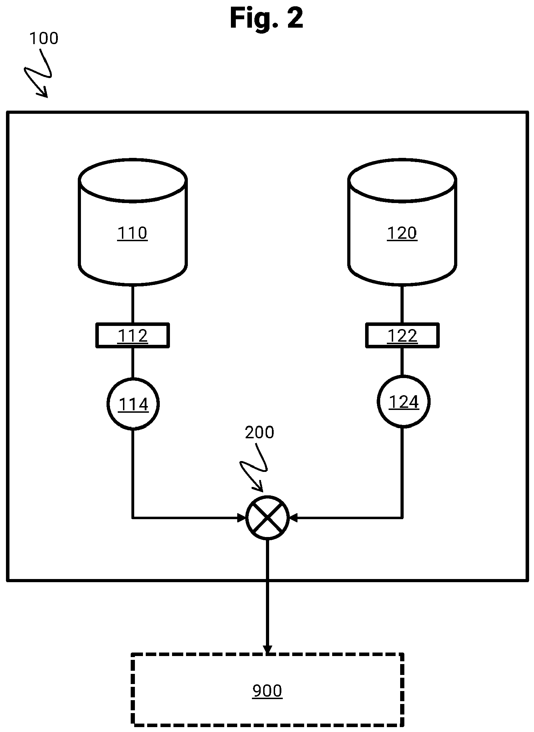

In Figure 2, a purge fluid generator similar to the one of Figure 1 is shown. The purge fluid generator further comprises a first adjustable valve 112 for controlling the gas flow from the first gas supply unit to the mixing unit 200 and a first sensor 114 for measuring the gas flow from the first gas supply unit to the mixing unit. The first adjustable valve 112 may be positioned either upstream or downstream of the first sensor 114. The purge fluid generator further comprises a second adjustable valve 122 for controlling the gas flow from the second gas supply unit to the mixing unit and a second sensor 124 for measuring the gas flow from the first gas supply unit to the mixing unit. The second adjustable valve 122 may be positioned either upstream or downstream of the second sensor 124.

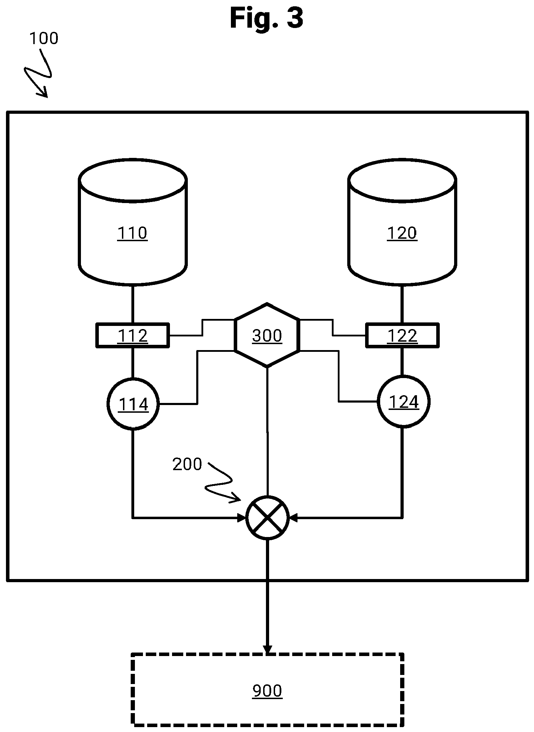

The purge fluid generator shown in Figure 3 is similar to the one of figure 2. The purge fluid generator further comprises a control unit 300. The control unit 300 is optionally connected to one or more of the first adjustable valve 112, the first sensor 114, the second adjustable valve 122, and the second sensor 124.

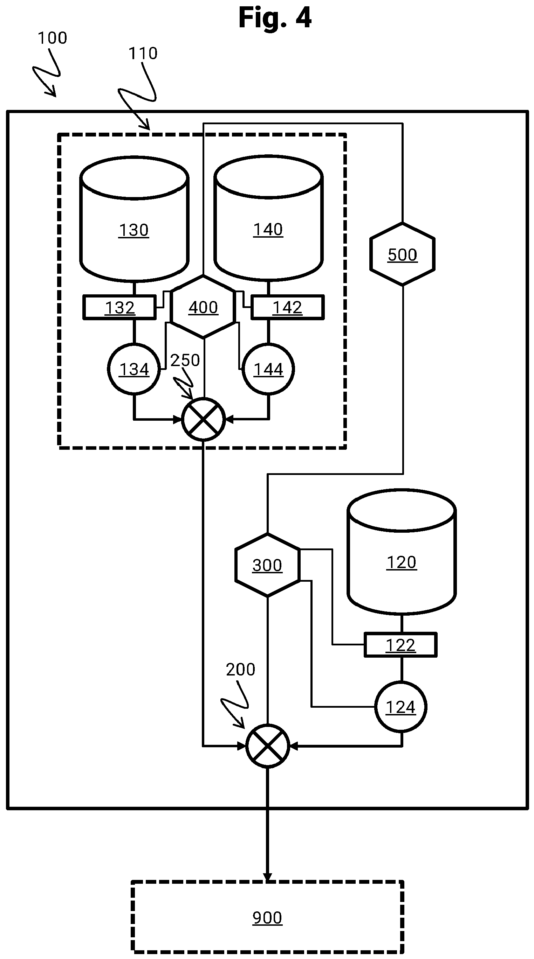

Figure 4 shows a variant of the purge fluid generator of Figure 3. In this example, the first gas supply unit 110 comprises a third gas supply unit 130, a fourth gas supply unit 140, a first gas supply outlet, and a second mixing unit 250. A first inlet of the second mixing unit 250 is connected to the third gas supply unit 130. A second inlet of the second mixing unit 250 is connected to the fourth gas supply unit 140. An outlet of the second mixing unit 250 is connected to the first gas supply outlet. The first gas supply outlet is connected to the first inlet of the mixing unit (200). The purge fluid generator further comprises a third adjustable valve 132 for controlling the gas flow from the third gas supply unit 130 to the second mixing unit 250 and a third sensor 134 for measuring the gas flow from the third gas supply unit to the second mixing unit 250. The third adjustable valve 132 may be positioned either upstream or downstream of the third sensor 134. The purge fluid generator further comprises a fourth adjustable valve 142 for controlling the gas flow from the fourth gas supply unit to the second mixing unit 250 and a fourth sensor 144 for measuring the gas flow from the fourth gas supply 140 unit to the second mixing unit 250. The fourth adjustable valve 142 may be positioned either upstream or downstream of the fourth sensor 144. Since the outlet gas flow from second mixing unit 250 can be measured through third sensor 132 and fourth sensor 142 and be controlled through third adjustable valve 134 and fourth adjustable valve 144, using a first sensor 112 and a first adjustable valve 114 is no longer necessary. The first gas supply unity has a control unit 400 which is connected to the third adjustable valve 132, the third sensor 134, the fourth adjustable valve 142, and the fourth sensor 144. The purge gas generator further comprises a control unit 500 which is connect to control unit 300 and control unit 400. The three control units may be designed as separate control units, may be pairwise combined into combined control units, or all three may be combined into one single control unit.

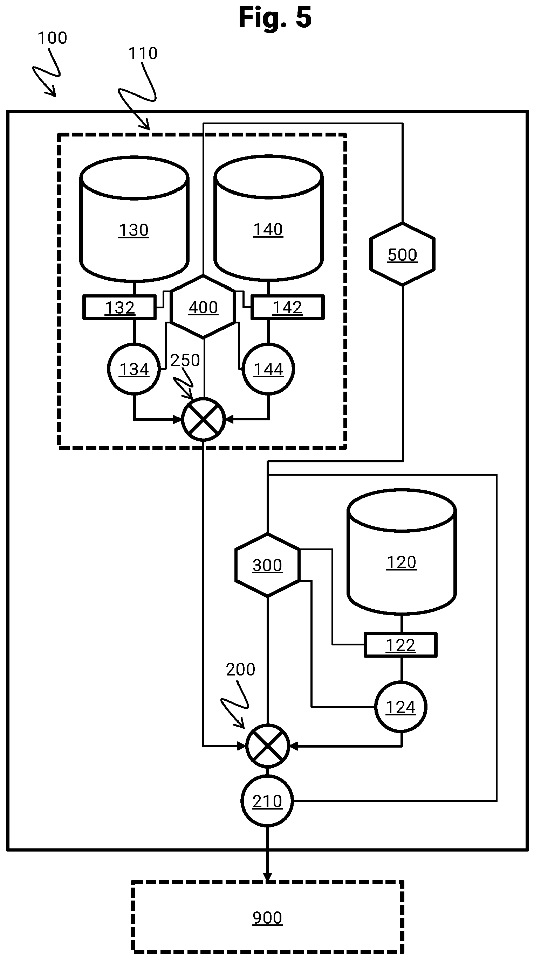

As can be seen in Figure 5, the purge fluid generator 100 may comprise a pressure or flow sensor 210 positioned at the outlet of mixing unit 200. The sensor 210 is connected to control unit 300 or to control unit 500 and allows the control unit to control the outlet pressure or flow through adjusting any or all of the adjustable valves in the purge fluid generator.

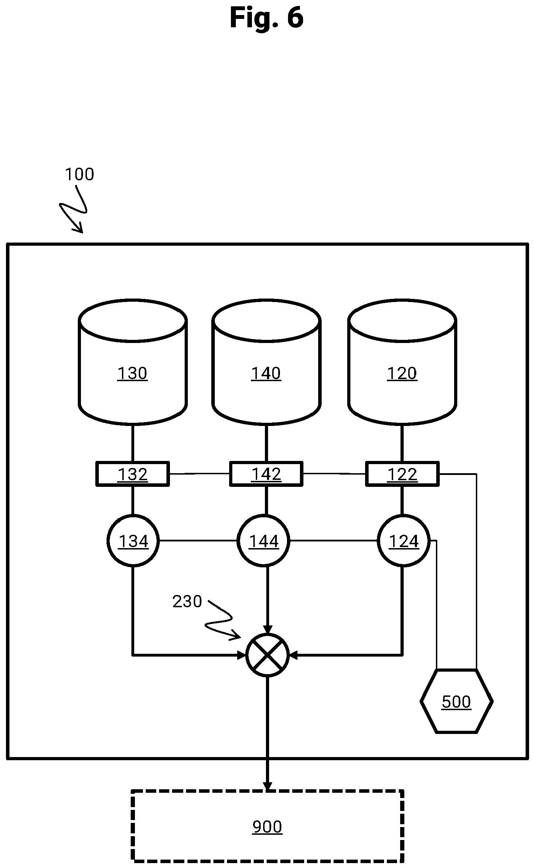

Figure 6 shows an alternative configuration of the purge fluid generator 100. The outlet of each of third gas supply unit 130, fourth gas supply unit 140, and second gas supply unit 120 are connected to one of the three inlets of the mixing unit 230. The outlet of mixing unit 230 is connected to the membrane oxygenation device 900 (not part of the invention). The purge fluid generator comprises a third adjustable valve 132 for controlling the gas flow from the third gas supply unit 130 to the mixing unit 230 and a third sensor 134 for measuring the gas flow from the third gas supply unit to the mixing unit 230. The third adjustable valve 132 may be positioned either upstream or downstream of the third sensor 134. The purge fluid generator further comprises a fourth adjustable valve 142 for controlling the gas flow from the fourth gas supply unit to the mixing unit 230 and a fourth sensor 144 for measuring the gas flow from the fourth gas supply 140 unit to the second unit 230. The purge fluid generator further comprises a second adjustable valve 122 for controlling the gas flow from the second gas supply unit to the mixing unit and a second sensor 124 for measuring the gas flow from the first gas supply unit to the mixing unit. The second adjustable valve 122 may be positioned either upstream or downstream of the second sensor 124. The purge fluid generator further comprises a control unit 500 which is connected to third adjustable valve 132, fourth adjustable valve 142, second adjustable valve 122, third sensor 134, fourth sensor 144, and second sensor 124. The control unit 500 may be configured to control the composition, the flow, and/or the pressure of the purge fluid at the outlet of the mixing unit 230 through adjusting the adjustable valves. An optional fifth gas supply unit may be connected to a fourth inlet of the mixing unit 230. The gas flow from the fifth gas supply unit may be measured with a fifth sensor and the gas flow may be controlled through a fifth adjustable valve.

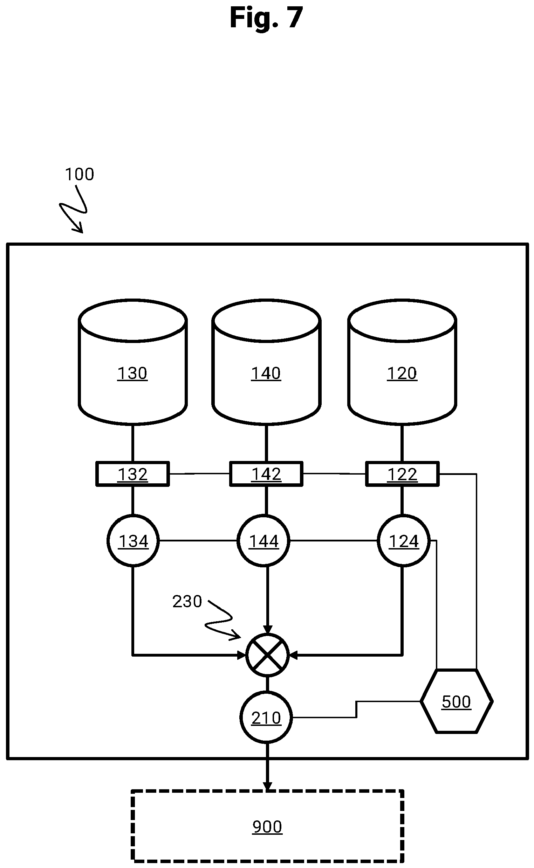

The purge fluid generator 100 of Figure 7 is similar to the purge fluid generator of Figure 6. The purge fluid generator of Figure 7 further comprises a pressure or flow sensor 210 positioned at the outlet of mixing unit 230. The sensor 210 is connected to control unit 500 and allows the control unit to control the outlet pressure or flow through adjusting any or all of the adjustable valves in the purge fluid generator.

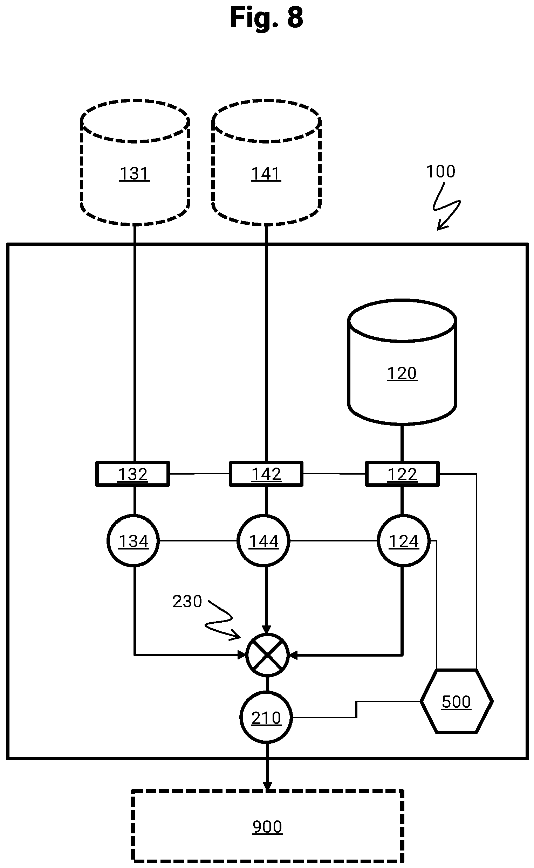

As can be seen in Figure 8, the third gas supply unit may be replaced with an external third gas supply unit 131 and the fourth gas supply unit may be replaced with an external fourth gas supply unit 131.

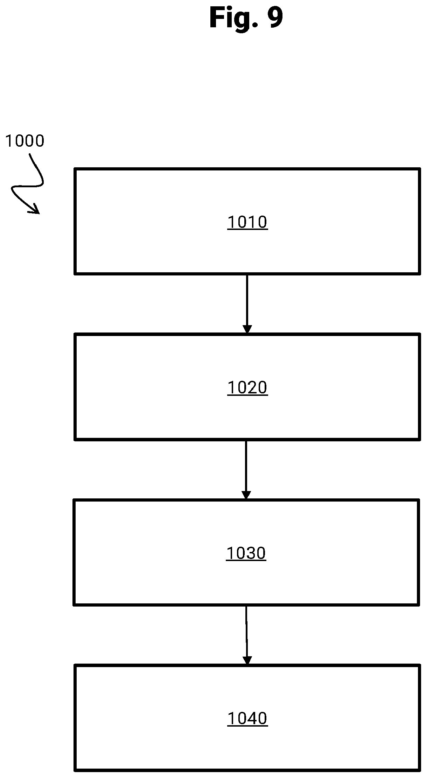

The method for conditioning the oxygenation membrane in extracorporeal membrane oxygenation 1000 according to the present invention is shown in Figure 9. The method 1000 comprises the step 1010 of receiving a purge fluid for membrane oxygenation comprising at least one of the following: oxygen, nitrogen, carbon dioxide, air. The method 1000further comprises the step 1020 of supplying a gas composition comprising nitric oxide. The method 1000 comprises the step 1030 of mixing the gas composition into the purge fluid for membrane oxygenation. The method 1000 further comprises the step 1040 of exposing one surface of the oxygenation membrane to the purge fluid mixed with the gas composition. The method 1000 may further comprise the step of monitoring the nitric oxide or nitrogen dioxide concentration in the oxygenated return blood of the extracorporeal membrane oxygenation apparatus in combination with the step of adjusting the nitric oxide concentration in the purge fluid in response to the nitric oxide or nitrogen dioxide concentration in the oxygenated return blood of the extracorporeal membrane oxygenation apparatus.

## Claims

1. Gas composition for use in preparation of a purge fluid for membrane oxygenation
**characterized in that**
the gas composition comprises at least 3'000 volume-PPM of nitric oxide.

2. The gas composition for use in preparation of a purge fluid for membrane oxygenation according to Claim 1
**wherein**
the balance of the gas composition is nitrogen.

3. A purge fluid generator (100) for supplying a purge fluid to a membrane oxygenation device (900)
comprising
• a first gas supply unit (110)
• a second gas supply unit (120)
• a purge fluid outlet
• a mixing unit (200) wherein
∘ a first inlet of the mixing unit is connected to the first gas supply unit
∘ a second inlet of the mixing unit is connected to the second gas supply unit
**characterized in that**
• the first gas supply unit is configured to supply a gas mixture comprising at least one gas selected from the group of air, oxygen, nitrogen, or carbon dioxide
• the second gas supply unit is configured to supply the gas composition for use in preparation of a purge fluid for membrane oxygenation according to Claim 1 or Claim 2
• the mixing unit is configured to deliver a purge fluid comprising between 1 and 200 volume-PPM of nitric oxide into the purge fluid outlet.

4. A purge fluid generator (100) for supplying a purge fluid to a membrane oxygenation device (900) according to Claim 3
**wherein the**
the purge fluid generator further comprises
• a first adjustable valve (112) for controlling the gas flow from the first gas supply unit to the mixing unit
• a first sensor (114) for measuring the gas flow from the first gas supply unit to the mixing unit
• a second adjustable valve (122) for controlling the gas flow from the second gas supply unit to the mixing unit
• a second sensor (124) for measuring the gas flow from the second gas supply unit to the mixing unit
**and wherein**
the purge fluid generator further comprises a control unit (300),
• the control unit being connected to the first flow sensor (114) for receiving a first signal indicative of the gas flow from the first gas supply unit to the mixing unit
• the control unit being connected to the second flow sensor (124) for receiving a second signal indicative of the gas flow from the second gas supply unit to the mixing unit
• the control unit being connected to the first valve (112) and the second valve (122)
**and wherein**
the control unit is configured to control the composition of the purge fluid by adjusting the first and the second adjustable valves.

5. A purge fluid generator for supplying purge fluid to a membrane oxygenation device according to any of Claim 3 through Claim 4
**wherein**
the first gas supply unit (110) comprises
• a third gas supply unit (130)
• a fourth gas supply unit (140)
• a first gas supply outlet
• a second mixing unit (250) wherein
∘ a first inlet of the second mixing unit is connected to the third gas supply unit
∘ a second inlet of the second mixing unit is connected to the fourth gas supply unit
∘ an outlet of the second mixing unit is connected to the first gas supply outlet
**and wherein**
the first gas supply outlet is connected to the first inlet of the mixing unit (200).

6. A purge fluid generator (100) for supplying purge fluid to a membrane oxygenation device (900) according to any of Claim 3 through Claim 5
**wherein**
the purge fluid generator further comprises a pressure sensor (210) for measuring the pressure of the pressure of the purge fluid at the outlet of the mixing unit (200).
**and wherein**
the pressure sensor is connected to the control unit (300)
**and wherein**
the control unit is configured to control the pressure of the purge fluid at the outlet of the mixing unit in response to the pressure sensed by the pressure sensor.

7. A purge fluid generator (100) for supplying purge fluid to a membrane oxygenation device (900) according to any of Claim 3 through Claim 6
**wherein**
the purge fluid generator further comprises a concentration sensor (210) for measuring the nitric oxide partial pressure in the purge fluid at the outlet of the mixing unit (200).
**and wherein**
the concentration sensor is connected to the control unit (300)
**and wherein**
the control unit is configured to control the nitric oxide partial pressure in the purge fluid at the outlet of the mixing unit in response to the concentration sensed by the concentration sensor.

8. A purge fluid generator (100) for supplying purge fluid to a membrane oxygenation device (900) according to any of Claim 3 through Claim 6
**wherein**
the control unit (300,500) is configured to receive one or more sensor signals indicative of
• oxygen partial pressure of the purge fluid flowing to the membrane oxygenation device
• carbon dioxide partial pressure of the purge fluid flowing to the membrane oxygenation device
• oxygen partial pressure of the purge fluid emitted from the membrane oxygenation device
• carbon dioxide partial pressure of the purge fluid emitted from the membrane oxygenation device
• oxygen partial pressure of the purge fluid flowing to a ventilator connected to the same patient as the membrane oxygenation device
• carbon dioxide partial pressure of the purge fluid flowing to the ventilator
• oxygen partial pressure of the purge fluid emitted from the ventilator
• carbon dioxide partial pressure of the purge fluid emitted from the ventilator
• water vapor partial pressure of the purge fluid emitted from the membrane oxygenation device
• nitric oxide or nitrogen dioxide in the oxygenated return blood of the extracorporeal membrane oxygenation apparatus
**and wherein**
the control unit (300, 500) is configured to control the composition of the purge fluid at the outlet of the mixing unit (210) or to display or play back an alarm signal in response to any of the sensor signals received.

9. A purge fluid generator (100) for supplying purge fluid to a membrane oxygenation device (900) according to any of Claim 3 through Claim 8
**wherein**
the second gas supply unit comprises a pressure vessel filled with the gas composition for use in preparation of a purge fluid for membrane oxygenation according to Claim 1 or Claim 2.

10. A purge fluid generator (100) for supplying purge fluid to a membrane oxygenation device (900) according to Claim 9
**wherein**
the volume of the pressure vessel is no more than 5 litres.

11. A purge fluid generator (100) for supplying purge fluid to a membrane oxygenation device (900) according to Claim 10
wherein
the purge fluid generator is configured for mobile use.

12. A pressure vessel for use in in a purge fluid generator (100) for supplying purge fluid to a membrane oxygenation device (900)
**wherein**
• the pressure vessel has a volume of less than 5 litres
• the pressure vessel contains the gas composition for use in preparation of a purge fluid for membrane oxygenation according to Claim 1 or Claim 2

13. A method for conditioning the oxygenation membrane in extracorporeal membrane oxygenation (1000)
comprising the steps of
• receiving a purge fluid for membrane oxygenation comprising at least one of the following: oxygen, nitrogen, carbon dioxide, air (1010)
• supplying a gas composition comprising nitric oxide (1020)
• mixing the gas composition into the purge fluid for membrane oxygenation (1030)
• exposing one surface of the oxygenation membrane to the purge fluid mixed with the gas composition (1040)

14. The method for conditioning the oxygenation membrane in extracorporeal membrane oxygenation (1000) according to Claim 13
**wherein**
the gas composition comprises at least 3'000 volume-PPM of nitric oxide

15. The method for conditioning the oxygenation membrane in extracorporeal membrane oxygenation (1000) according to Claim 14
**wherein**
the method further comprises the steps of
• monitoring the nitric oxide or nitrogen dioxide concentration in the oxygenated return blood of the extracorporeal membrane oxygenation apparatus
• adjusting the nitric oxide concentration in the purge fluid in response to the nitric oxide or nitrogen dioxide concentration in the oxygenated return blood of the extracorporeal membrane oxygenation apparatus
